# EUROPEAN PATENT SPECIFICATION

(11) **EP 4 017 847 B1**
(45) Date of publication and mention of the grant of the patent: **01.10.2025**
(21) Application number: 20765410.4
(22) Date of filing: 19.08.2020
(51) Int. Cl.: C07D 401/04

(54) **PROCESS FOR THE PREPARATION OF CARBOXYLIC ACID DERIVATIVES OF 3-BROMO-4,5-DIHYDRO-1H-PYRAZOLES**
VERFAHREN ZUR HERSTELLUNG VON CARBOXYLSÄUREDERIVATEN VON 3-BROM-4,5-DIHYDRO-1H-PYRAZOLEN
PROCÉDÉ DE PRÉPARATION DE DÉRIVÉS D'ACIDE CARBOXYLIQUE DE 3-BROMO-4,5-DIHYDRO-1H-PYRAZOLES

(30) Priority: 19.08.2019 US 201962888667 P; 22.08.2019 US 201962890154 P
(43) Date of publication of application: 29.06.2022
(73) Proprietor: FMC CORPORATION, Philadelphia, PA 19104 (US); FMC IP Technology GmbH, 8212 Neuhausen am Rheinfall (CH)
(72) Inventor: MAO, Jianhua, Philadelphia, PA 19104 (US); MA, Jingyi, Philadelphia, PA 19104 (US); PENG, Dongjie, Philadelphia, PA 19104 (US); HUO, Chunyan, Philadelphia, PA 19104 (US)
(74) Representative: Dehns
(86) International application number: PCT/US2020/046947
(87) International publication number: WO 2021/034904

(56) References cited:
- WO-A1-03/016283
- CN-A- 102 399 211

## Description

### FIELD

This disclosure relates to the preparation of 3-bromo-4,5-dihydro-1*H*-pyrazoles using a novel one-step bromination process. Compounds prepared by the process disclosed herein are useful for preparation of certain anthranilamide compounds that are of interest as insecticides.

### BACKGROUND

Ethyl 3-bromo-1-(3-chloropyridin-2-yl)-4,5-dihydro-1H-pyrazole-5-carboxylate is an intermediate in the preparation of anthranilamides, such as for example the insecticides chlorantraniliprole and cyantraniliprole. Preparation of ethyl 3-bromo-1-(3-chloropyridin-2-yl)-4,5-dihydro-1H-pyrazole-5-carboxylate from ethyl 1-(3-chloropyridin-2-yl)-3-hydroxy-4,5-dihydro-1H-pyrazole-5-carboxylate in a two-step process has been described in US Pat. No. 6,965,032 to DuPont. In the first step, ethyl 1-(3-chloropyridin-2-yl)-3-hydroxy-4,5-dihydro-1H-pyrazole-5-carboxylate is treated with benzenesulfonyl chloride to give ethyl 1-(3-chloropyridin-2-yl)-3-((phenylsulfonyl)oxy)-4,5-dihydro-1*H*-pyrazole-5-carboxylate. In the second step, ethyl 1-(3-chloropyridin-2-yl)-3-((phenylsulfonyl)oxy)-4,5-dihydro-1H-pyrazole-5-carboxylate is treated with a solution of hydrogen bromide in acetic acid, resulting in an overall yield of 86% of Ethyl 3-bromo-1-(3-chloropyridin-2-yl)-4,5-dihydro-1*H*-pyrazole-5-carboxylate. Alternative processes for the preparation of ethyl 3-bromo-1-(3-chloropyridin-2-yl)-4,5-dihydro-1H-pyrazole-5-carboxylate from ethyl 1-(3-chloropyridin-2-yl)-3-hydroxy-4,5-dihydro-1H-pyrazole-5-carboxylate, such as a one-step bromination process using Phosphorus oxybromide (POBr₃) or Phosphorus pentabromide (PBr₅), have also been described. POBr₃ and PBr₅, however, are not commercially available on a scale necessary for production of anthranilamide compounds. While PBr₅ is potentially the least expensive to produce, it has the tendency to agglomerate into a hard, solid block, making shipping and storing it difficult. One way to circumvent this problem is by preparing PBr₅ in situ, by reacting PBr₃ with bromine (Pat. Application No. CN102399211A). Both, PBr3 and bromine, are commercially available at sufficient scale. In this option PBr₃ is treated with bromine, resulting in a slurry of PBr₅ to which ethyl 1-(3-chloropyridin-2-yl)-3-hydroxy-4,5-dihydro-1H-pyrazole-5-carboxylate is added. However, one of the drawbacks of this method is that PBr₅ tends to coat the walls of the reactor as it is being formed, which makes the transfer of the mixture to another reactor challenging.

The present disclosure provides a novel process useful for preparing ethyl 3-bromo-1-(3-chloropyridin-2-yl)-4,5-dihydro-1*H*-pyrazole-5-carboxylate and derivatives thereof in a convenient and cost-effective way.

### SUMMARY

Provided herein is a method of preparing a compound of Formula (I) 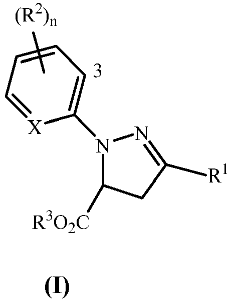 wherein R¹ is bromo;
each R² is independently C₁-C₄ alkyl, C₂-C₄ alkenyl, C₂-C₄ alkynyl, C₃-C₆ cycloalkyl, C₁-C₄ haloalkyl, C₂-C₄ haloalkenyl, C₂-C₄ haloalkynyl, C₃-C₆ halocycloalkyl, halogen, CN, NO₂, C₁-C₄ alkoxy, C₁-C₄ haloalkoxy, C₁-C₄ alkylthio, C₁-C₄ alkylsulfinyl, C₁-C₄ alkylsulfonyl, C₁-C₄ alkylamino, C₂-C₈ dialkylamino, C₃-C₆ cycloalkylamino, C₃-C₆ (alkyl)cycloalkylamino, C₂-C₄ alkylcarbonyl, C₂-C₆ alkoxycarbonyl, C₂-C₆ alkylaminocarbonyl, C₃-C₈ dialkylaminocarbonyl or C₃-C₆ trialkylsilyl;
R³ is H or C₁-C₄ alkyl;
X is N or CR₄;
R⁴ is H or R²; and
n is 0, 1, 2, or 3, with the proviso that when X is CH then n is at least 1, the method comprising (1) treating a compound of Formula (A) 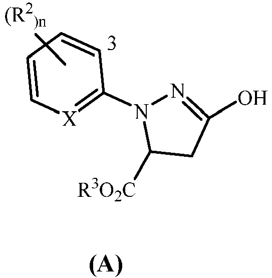 wherein X, R², and n are as described above for Formula (I) and R³ is C₁-C₄ alkyl;
   with phosphorus tribromide (PBr₃); and (2) treating the resulting product with bromine (Br₂); and when preparing compounds of Formula (I) wherein R³ is H (3) converting the compound formed in (2) to a compound wherein R³ is H. Also disclosed is a method of preparing a compound of Formula (II), 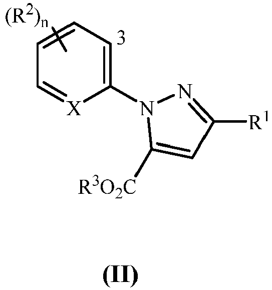
wherein R¹ is halogen (and X, R², R³ and n are defined as above for Formula (I). The method is characterized by (4) treating a Compound of Formula (A) according to the steps recited above for forming a compound of Formula (I), and oxidizing the compound of Formula (I); and when a compound of Formula (I) wherein R³ is C₁-C₄ alkyl is used to prepare a compound of Formula **(II)** wherein R³ is H, (5) converting the compound formed in (3) to a compound of Formula **(II)** wherein R³ is H.

### DETAILED DESCRIPTION

In the above recitations, the term "alkyl", used either alone or in compound words such as "alkylthio" or "haloalkyl" includes straight-chain or branched alkyl, such as methyl, ethyl, n-propyl, i-propyl, or the different butyl, pentyl or hexyl isomers. "Alkenyl" can include straight-chain or branched alkenes such as 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as 1-propynyl, 2-propynyl and the different butynyl, pentynyl and hexynyl isomers. "Alkynyl" can also include moieties comprised of multiple triple bonds such as 2,5-hexadiynyl. "Alkoxy" includes, for example, methoxy, ethoxy, n-propyloxy, isopropyloxy and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. "Alkylthio" includes branched or straight-chain alkylthio moieties such as methylthio, ethylthio, and the different propylthio, butylthio, pentylthio and hexylthio isomers. "Cycloalkyl" includes, for example, cyclopropyl, cyclobutyl, cyclopentyl and cyclohexyl. "Cycloalkylalkyl" indicates an alkyl group substituted with a cycloalky group and includes, for example, cyclopropylmethyl, cyclobutylethyl, cyclopentylpropyl and cyclohexylmethyl. "Cycloalkylamino" means the amino nitrogen atom is attached to a cycloalkyl radical and a hydrogen atom and includes groups such as cyclopropylamino, cyclobutylamino, cyclopentylamino and cyclohexylamino. "(Alkyl)cycloalkylamino" means a cycloalkylamino group where the hydrogen atom is replaced by an alkyl radical; examples include groups such as **(alkyl)cyclopropylamino,** (alkyl)cyclobutylamino, (alkyl)cyclopentylamino and (alkyl)cyclohexylamino. Preferably the alkyl in (alkyl)cycloalkylamino is C₁-C₄ alkyl, while the cycloalkyl in cycloalkylamino and (alkyl)cycloalkylamino is C₃-C₆ cycloalkyl.

The term in this application "aryl" refers to an aromatic ring or ring system or a heteroaromatic ring or ring system, each ring or ring system optionally substituted. The term "aromatic ring system" denotes fully unsaturated carbocycles and heterocycles in which at least one ring of a polycyclic ring system is aromatic. Aromatic indicates that each of ring atoms is essentially in the same plane and has a *p*-orbital perpendicular to the ring plane, and in which (4n + 2) π electrons, when n is 0 or a positive integer, are associated with the ring to comply with Hückel's rule. The term "aromatic carbocyclic ring system" includes fully aromatic carbocycles and carbocycles in which at least one ring of a polycyclic ring system is aromatic (e.g. phenyl and naphthyl). The term "heteroaromatic ring or ring system" includes fully aromatic heterocycles and heterocycles in which at least one ring of a polycyclic ring system is aromatic and in which at least one ring atom is not carbon and can contain 1 to 4 heteroatoms independently selected from the group consisting of nitrogen, oxygen and sulfur, provided that each heteroaromatic ring contains no more than 4 nitrogens, no more than 2 oxygens and no more than 2 sulfurs (where aromatic indicates that the Hückel rule is satisfied). The heterocyclic ring systems can be attached through any available carbon or nitrogen by replacement of a hydrogen on said carbon or nitrogen. More specifically, the term "aryl" refers to the moiety 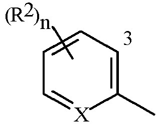 wherein R² and n are defined as above and the "3" indicates the 3-position for substituents on the moiety.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different. Examples of "haloalkyl" include F₃C, ClCH₂, CF₃CH₂ and CF₃CCl₂. The terms "haloalkenyl", "haloalkynyl", "haloalkoxy", and the like, are defined analogously to the term "haloalkyl". Examples of "haloalkenyl" include (Cl)₂C=CHCH₂ and CF₃CH₂CH=CHCH₂. Examples of "haloalkynyl" include HC≡CCHCl, CF₃C≡C, CCl₃C≡C and FCH₂C≡CCH₂. Examples of "haloalkoxy" include CF₃O, CCl₃CH₂O, HCF₂CH₂CH₂O and CF₃CH₂O.

Examples of "alkylcarbonyl" include C(O)CH₃, C(O)CH₂CH₂CH₃ and C(O)CH(CH₃)₂. Examples of "alkoxycarbonyl" include CH₃OC(=O), CH₃CH₂OC(=O), CH₃CH₂CH₂OC(=O), (CH₃)₂CHOC(=O) and the different butoxy- or pentoxycarbonyl isomers. The terms "alkylaminocarbonyl" and "dialkylaminocarbonyl" include, for example, CH₃NHC(=O), CH₃CH₂NHC(=O) and (CH₃)₂NC(=O).

The total number of carbon atoms in a substituent group is indicated by the "Cᵢ-Cⱼ" prefix where i and j are numbers from 1 to 8. For example, C₁-C₃ alkylsulfonyl designates methylsulfonyl through propylsulfonyl. In the above recitations, when a compound of Formula **(I)** contains a heteroaromatic ring, all substituents are attached to this ring through any available carbon or nitrogen by replacement of a hydrogen on said carbon or nitrogen.

When a group contains a substituent which can be hydrogen, for example R⁴, then, when this substituent is taken as hydrogen, it is recognized that this is equivalent to said group being unsubstituted.

Certain compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. Accordingly, the embodiments of this disclosure include:
Embodiment 1. A method of preparing a compound of Formula **(I)** wherein n is 1, 2, or 3.
Embodiment 2. The method according to Embodiment 1 wherein n is 1.
Embodiment 5. The method according to Embodiments 1 to 4 wherein each R² is independently Cl or Br, and one R² is at the 3-position.
Embodiment 6. The method according to Embodiments 1 to 5 wherein
   each R² is Cl at the 3-position.
Embodiment 7. The method according to Embodiments 1 to 6 wherein R³ is C₁-C₄ alkyl.
Embodiment 8. The method according to Embodiments 1 to 7 wherein R³ is Et.
Embodiment 9. The method according to Embodiments 1 to 8 wherein and X is N.
Embodiment 10. The method of Embodiments 1-10, wherein the method is carried out in the presence of a solvent.
Embodiment 11. The method of Embodiment 10 wherein the solvent is selected from acetonitrile, N,N-dimethylformamide, dimethylacetamide, chloroform, acetone, propionitrile, chlorobenzene, tetrachloromethane, dichlorobenzene, dichloromethane, and 1,2-dichloroethane.
Embodiment 12. The method of Embodiment 11 wherein the solvent is selected from acetonitrile, chlorobenzene, dichloromethane, and 1,2-dichloroethane.
Embodiment 13. The method of Embodiment 11 or 12 wherein the solvent is acetonitrile.
Embodiment 14. The method of any one of Embodiments 1-13, further comprising treating the product after step (2) with a base.
Embodiment 15. The method of any one of Embodiments 1-14, wherein the base is selected from solid sodium bicarbonate, sodium bicarbonate, potassium bicarbonate, calcium bicarbonate, sodium carbonate, potassium carbonate, ammonium carbonate, ammonium bicarbonate, trisodium phosphate, tripotassium phosphate, caesium carbonate, triethylamine, pyridine, N-methylimidazole, potassium hydrogen phosphate, sodium hydrogen phosphate, sodium hydroxide, and potassium hydroxide.
Embodiment 16. The method of any one of Embodiments 14-15 wherein the base is selected from solid sodium bicarbonate, potassium carbonate, and sodium carbonate.
Embodiment 17. The method of any one of Embodiments 14-16 wherein the base is solid sodium bicarbonate.
Embodiment 18. The method of Embodiment 17 further comprising adding water after treating the product with solid sodium bicarbonate.
Embodiment 19. The method of any one of Embodiments 14- 16 wherein the base is a saturated sodium bicarbonate solution.
Embodiment 20. The method of Embodiment 19 further comprising adding solid sodium bicarbonate after treating the product with the saturated sodium bicarbonate solution.
Embodiment 21. The method of any one of Embodiments 14-16 wherein the base is a sodium carbonate solution.
Embodiment 22. The method of any one of Embodiments 14-16 wherein the base is a potassium carbonate solution.
Embodiment 23. The method any one of Embodiments 1-22, further comprising isolating a compound of Formula (I).
Embodiment 24. The method of any one of Embodiments 1-23 wherein the compound of Formula (I) is ethyl 3-bromo-1-(3-chloropyridin-2-yl)-4,5-dihydro-1H-pyrazole-5-carboxylate.
Embodiment 25. A method of preparing a compound of Formula **(II)**
   wherein n is 1, 2, or 3.
Embodiment 27. The method of Embodiment 25 wherein R² is independently Cl or Br.
Embodiment 28. The method of Embodiment 27 wherein one R² is at the 3-position.
Embodiment 29. The method of any one of Embodiments 25-28 wherein R³ is C₁-C₄ alkyl.
Embodiment 30. The method of any one of Embodiments 25-29 wherein X is N.
Embodiment 31. The method of any one of Embodiments 25-30 further comprising isolating the compound of Formula (II).
Embodiment 32. The method of any one of Embodiments 25-30 wherein the compound of Formula **(II)** is ethyl 3-bromo-1-(3-chloro-2-pyridinyl)-1H-pyrazole-5-carboxylate,
Embodiment 33. The method of any one of Embodiments 1-32, wherein the steps (1) and (2) are independently carried out at the temperature from 15°C to 50°C. In one embodiment useful temperatures at which steps (1) and (2) can be carried out include 15 °C, 20 °C, 25 °C, 30 °C, 35 °C, 40 °C, 45 °C, and 50 °C.
Embodiment 34. The method of any one of Embodiments 1-33 wherein the compound of Formula (I) is ethyl 3-bromo-1-(3-chloropyridin-2-yl)-4,5-dihydro-1H-pyrazole-5-carboxylate, having the following structure: 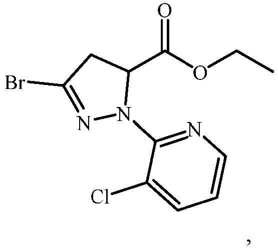 and the compound of Formula (A) is ethyl 1-(3-chloropyridin-2-yl)-3-hydroxy-4,5-dihydro-1H-pyrazole-5-carboxylate, having the structure:

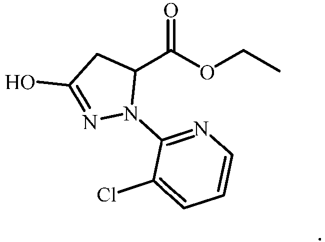

A stepwise process of preparing compounds of Formula **(I)** and Formula **(II)** is described below.

Processes for the preparation of a compound of Formula (A) are known in the art and have been described for example in US 6,965,032 B2.

The process for preparing a compound of Formula (I) and Formula **(II)** provided herein comprises (1) treating a compound of Formula (A) 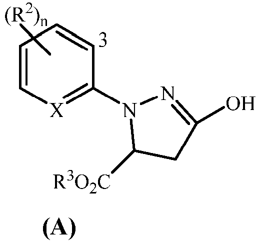 wherein X, R² and n are as defined as above and R³ is H; or R³ is C₁-C₄ alkyl with PBr₃ in presence of a solvent. The resulting product is then (2) treated with bromine to form a compound of Formula (I); 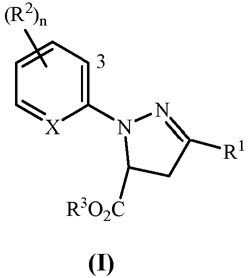 wherein X, R¹, R² and n are as defined as above and R³ is H; or R³ is C₁-C₄ alkyl.

Due to excess bromine, an amount of a compound of Formula (I) is oxidized to form a compound of Formula **(II)** 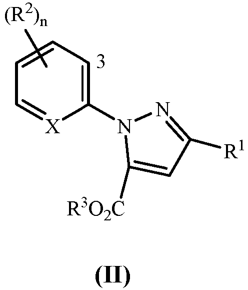 wherein X, R¹, R² and n are as defined as above and R³ is H; or R₃ is C₁-C₄ alkyl.

Scheme 1 illustrates steps (1) and (2) in more detail.
A compound of Formula (A) is first (1) treated with PBr₃ in the presence of a solvent. Useful solvents include, but are not limited to acetonitrile, N,N-dimethylformamide, dimethylacetamide, chloroform, acetone, propionitrile, chlorobenzene, tetrachloromethane, dichlorobenzene, dichloromethane or 1,2-dichloroethane. In one embodiment, the solvent is selected from acetonitrile, chlorobenzene, dichloromethane, and 1,2-dichloroethane. In one embodiment, acetonitrile is used as a solvent. The resulting product is then (2) treated with bromine, resulting in a slurry of a hydrobromide salt of a compound of Formula (I).

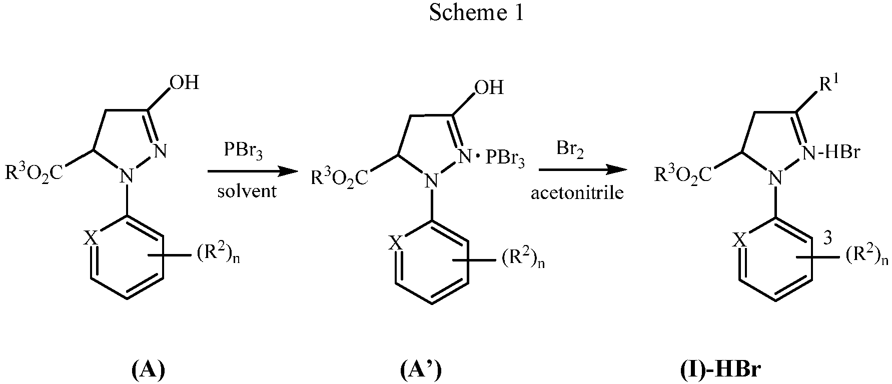

The reaction mass is then treated with an inorganic base, including, but not limited to sodium bicarbonate, potassium bicarbonate, calcium bicarbonate, sodium carbonate, potassium carbonate, ammonium carbonate, ammonium bicarbonate, trisodium phosphate, tripotassium phosphate, caesium carbonate, triethylamine, pyridine, N-methylimidazole, potassium hydrogen phosphate, sodium hydrogen phosphate, sodium hydroxide, or potassium hydroxide. In one embodiment the reaction mass is treated with solid sodium bicarbonate, followed by the addition of water to dissolve the solids. In one embodiment, the reaction mass is treated with saturated sodium bicarbonate solution, followed by solid sodium bicarbonate to complete neutralization.
In one embodiment, the reaction mass is neutralized by adding a sodium carbonate solution or a potassium carbonate solution.

Optionally, the desired product, a compound of Formula (I), can be isolated by methods known to those skilled in the art, including, but not limited to crystallization, extraction and distillation. Using the process disclosed herein, a yield of from about 90-95% of a compound of Formula (I) is achieved. It is noteworthy that in addition to a compound of Formula (I), the described process yields from about 1% to 5% of a compound of Formula **(II)**. It should be noted that this process circumvents formation of PBr₅.

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. Percentages are by weight except for chromatographic solvent mixtures or where otherwise indicated. Parts and percentages for chromatographic solvent mixtures are by volume unless otherwise indicated. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

¹H NMR spectra are reported in ppm downfield from tetramethylsilane; "s" means singlet, "d" means doublet, "t" means triplet, "q" means quartet, "m" means multiplet, "dd" means doublet of doublets, "dt" means doublet of triplets, and "br s" means broad singlet. All reagents described in the following examples are available from commercial sources.

### EXAMPLE 1

### Preparation of ethyl 3-bromo-1-(3-chloropyridin-2-yl)-4,5-dihydro-1H-pyrazole-5-carboxylate* and ethyl 3-bromo-1-(3-chloro-2-pyridinyl)-1H-pyrazole-5-carboxylate**using solid sodium bicarbonate for neutralization

150 g of ethyl 1-(3-chloropyridin-2-yl)-3-hydroxy-4,5-dihydro-1H-pyrazole-5-carboxylate was treated with 90.3 g phosphorus tribromide (PBr₃) in 750 ml acetonitrile at 30-50 °C for 0.5 hr. The mixture is then treated with 53.3 g Bromine (Br₂) at 30-40 °C. The reaction mass was heated to reflux (at about 83 °C, this can be carried out at about 80 °C to at about 83 °C) for 1-2 hrs. After the reaction was complete, the resulting ethyl 3-bromo-1-(3-chloropyridin-2-yl)-4,5-dihydro-1*H*-pyrazole-5-carboxylate-HBr slurry was treated portionwise with 140 g solid sodium bicarbonate. The mixture was then diluted by portionwise adding 600 g water. The two-phased mixture was adjusted to pH 7-8 and the phases were separated. The organic phase was concentrated to remove acetonitrile under reduced pressure. The residue was mixed with 120 g EtOH and 150 g water. The resulting slurry was filtered to afford 173.7g ethyl 3-bromo-1-(3-chloropyridin-2-yl)-4,5-dihydro-1*H-*pyrazole-5-carboxylate (98.1 wt.%), corresponding to a yield of 92%. Due to presence of excess bromine some of the ethyl 3-bromo-1-(3-chloropyridin-2-yl)-4,5-dihydro-1*H-*pyrazole-5-carboxylate was further oxidized, resulting in the generation of ethyl 3-Bromo-1-(3-chloro-2-pyridinyl)-1H-pyrazole-5-carboxylate, corresponding to a yield of about 1.0%-5.0%.

It should be noted that neutralizations with solid bicarbonate frequently do not go to completion due to encapsulation of the sodium bicarbonate with reaction products. Addition of a phase transfer catalyst is often required. Surprisingly, in this case the neutralization reaction proceeds to completion without a phase transfer catalyst. Accordingly, it is expected that any process producing ethyl 3-bromo-1-(3-chloropyridin-2-yl)-4,5-dihydro-1H-pyrazole-5-carboxylate-HBr slurry would similarly benefit from the addition of solid bicarbonate as disclosed herein.

### EXAMPLE 2 (Comparative, not claimed)

### Preparation of ethyl 3-bromo-1-(3-chloropyridin-2-yl)-4,5-dihydro-1H-pyrazole-5-carboxylate" using PBrS prepared in situ

In this Example PBr5 was prepared *in situ,* by reacting PBr3 with bromine. Ethyl 1-(3-chloropyridin-2-yl)-3-hydroxy-4,5-dihydro-1*H*-pyrazole-5-carboxylate is subsequently added to the PBr5 slurry.
Specifically, 81 g of PBr3 and 48 g of Br2 were mixed in 1490 mL acetonitrile at 20 °C for 2 hrs. The resulting PBr5 slurry was then treated with 68 g ethyl 1-(3-chloropyridin-2-yl)-3-hydroxy-4,5-dihydro-1*H*-pyrazole-5-carboxylate and the mixture was heated to 80 °C for 2 hrs. After the reaction was complete, the solvent was removed under pressure. The residue was dissolved in dichloromethane and the solution washed with water. The organic phase was concentrated to afford 82.1 g ethyl 3-bromo-1-(3-chloropyridin-2-yl)-4,5-dihydro-1*H-*pyrazole-5-carboxylate (90 wt%), corresponding to a yield of 88%.

### EXAMPLE 3

### Preparation of ethyl 3-bromo-1-(3-chloropyridin-2-yl)-4,5-dihydro-1H-pyrazole-5-carboxylate* and ethyl 3-bromo-1-(3-chloro-2-pyridinyl)-1H-pyrazole-5-carboxylate**using a sodium bicarbonate solution and solid sodium bicarbonate for neutralization

135 g of ethyl 1-(3-chloropyridin-2-yl)-3-hydroxy-4,5-dihydro-1*H*-pyrazole-5-carboxylate was treated with 74.4 g phosphorus tribromide (PBr₃) in 675 ml acetonitrile at 30-50 °C for 0.5 hr. The mixture is then treated with 43.9 g bromine (Br₂) at 30-40 °C. The reaction mass was heated to reflux (at about 83 °C) for 1-2 hrs. After the reaction was complete, the resulting ethyl 3-bromo-1-(3-chloropyridin-2-yl)-4,5-dihydro-1*H*-pyrazole-5-carboxylate-HBr slurry was treated portionwise with 720 g sodium bicarbonate solution in water (50 g solid sodium bicarbonate was dissolved in 675 g water), then another 65 g solid sodium bicarbonate was added portionwise. The two-phased mixture was adjusted to pH 7-8 and the phases were separated. The organic phase was concentrated to remove acetonitrile under reduced pressure. The residue was mixed with 106 g EtOH and 135 g water. The resulting slurry was filtered to afford 155 g ethyl 3-bromo-1-(3-chloropyridin-2-yl)-4,5-dihydro-1*H-*pyrazole-5-carboxylate (98.0 wt.%), corresponding to a yield of 91%. Due to presence of excess bromine some of the ethyl 3-bromo-1-(3-chloropyridin-2-yl)-4,5-dihydro-1*H-*pyrazole-5-carboxylate was further oxidized, resulting in the generation of ethyl 3-Bromo-1-(3-chloro-2-pyridinyl)-1H-pyrazole-5-carboxylate, corresponding to a yield of about 1.0%-5.0%.

### EXAMPLE 4

### Preparation of ethyl 3-bromo-1-(3-chloropyridin-2-yl)-4,5-dihydro-1H-pyrazole-5-carboxylate* and ethyl 3-bromo-1-(3-chloro-2-pyridinyl)-1H-pyrazole-5-carboxylate**using a sodium carbonate solution and solid sodium carbonate for neutralization

135 g of ethyl 1-(3-chloropyridin-2-yl)-3-hydroxy-4,5-dihydro-1*H*-pyrazole-5-carboxylate was treated with 74.4 g phosphorus tribromide (PBr₃) in 675 ml acetonitrile at 30-50 °C for 0.5 hr. The mixture is then treated with 43.9 g bromine (Br₂) at 30-40 °C. The reaction mass was heated to reflux (at about 83 °C) for 1-2 hrs. After the reaction was complete, the resulting ethyl 3-bromo-1-(3-chloropyridin-2-yl)-4,5-dihydro-1*H*-pyrazole-5-carboxylate-HBr slurry was treated portionwise with 748 g sodium carbonate solution in water (73 g solid sodium carbonate was dissolved in 675 g water). The two-phased mixture was adjusted to pH 7-8 and the phases were separated. The organic phase was concentrated to remove acetonitrile under reduced pressure. The residue was mixed with 106 g EtOH and 135 g water. The resulting slurry was filtered to afford 152 g ethyl 3-bromo-1-(3-chloropyridin-2-yl)-4,5-dihydro-1H-pyrazole-5-carboxylate (98.5 wt.%), corresponding to a yield of 90%. Due to presence of excess bromine some of the ethyl 3-bromo-1-(3-chloropyridin-2-yl)-4,5-dihydro-1H-pyrazole-5-carboxylate was further oxidized, resulting in the generation of ethyl 3-Bromo-1-(3-chloro-2-pyridinyl)-1H-pyrazole-5-carboxylate, corresponding to a yield of about 1.0%-5.0%.

### EXAMPLE 5

### Preparation of ethyl 3-bromo-1-(3-chloropyridin-2-yl)-4,5-dihydro-1H-pyrazole-5-carboxylate* and ethyl 3-bromo-1-(3-chloro-2-pyridinyl)-1H-pyrazole-5-carboxylate**using a potassium carbonate solution and solid potassium carbonate for neutralization

135 g of ethyl 1-(3-chloropyridin-2-yl)-3-hydroxy-4,5-dihydro-1*H*-pyrazole-5-carboxylate was treated with 74.4 g phosphorus tribromide (PBr₃) in 675 ml acetonitrile at 30-50 °C for 0.5 hr. The mixture is then treated with 43.9 g bromine (Br₂) at 30-40 °C. The reaction mass was heated to reflux (at about 83 °C) for 1-2 hrs. After the reaction was complete, the resulting ethyl 3-bromo-1-(3-chloropyridin-2-yl)-4,5-dihydro-1*H*-pyrazole-5-carboxylate-HBr slurry was treated portionwise with 768 g potassium carbonate solution in water (93 g solid potassium carbonate was dissolved in 675 g water). The two-phased mixture was adjusted to pH 7-8 and the phases were separated. The organic phase was concentrated to remove acetonitrile under reduced pressure. The residue was mixed with 106 g EtOH and 135 g water. The resulting slurry was filtered to afford 155 g ethyl 3-bromo-1-(3-chloropyridin-2-yl)-4,5-dihydro-1*H*-pyrazole-5-carboxylate (98.3 wt.%), corresponding to a yield of 92%. Due to presence of excess bromine some of the ethyl 3-bromo-1-(3-chloropyridin-2-yl)-4,5-dihydro-1*H*-pyrazole-5-carboxylate was further oxidized, resulting in the generation of ethyl 3-Bromo-1-(3-chloro-2-pyridinyl)-1H-pyrazole-5-carboxylate, corresponding to a yield of about 1.0%-5.0%.
* ¹H NMR (DMSO-d6, 400 MHz) δ = 1.12 (t, *J=* 7.0 Hz, 3H), 3.24-3.31 (m, 1H), 3.54-3.61 (m, 1H), 4.08 (q, *J=* 7.0 Hz, 2H), 5.14-5.19 (m, 1H), 6.98 (dd, *J= 4.8* Hz, *J=* 7.6 Hz, 1H), 7.83 (d, *J* = 7.7 Hz, 1H), 8.10 (d, *J =* 4.4 Hz, 1H).
**¹H NMR ((DMSO-d6, 400 MHz) δ = 1.20 (t, J = 7.2 Hz, 3H), 4.23 (q, *J =* 7.2 Hz, 2H), 6.94 (s, 1H), 7.44 (dd, *J=* 4.8 Hz, *J= 8.1* Hz, 1H), 7.91 (d, *J=* 8.1 Hz, 1H), 8.51 (d, *J= 4.8* Hz, 1H).

By the procedures described herein together with methods known in the art, the following compounds of Tables 1 and 2 can be prepared. The following abbreviations are used in the Tables: *t* is tertiary, s is secondary, *n* is normal, *i* is iso, Me is methyl, Et is ethyl, Pr is propyl, *i*-Pr is isopropyl and t-Bu is tertiary butyl.

**TABLE 1**

| 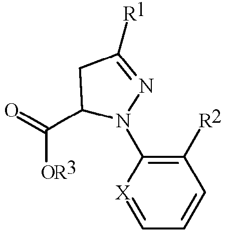 | | | | | | | | | | | | | | | |
|---|---|---|---|---|---|---|---|---|---|---|---|---|---|---|---|
| R¹ is Br | | | | | | | | | | | | | | | |
| X is N | | | | X is CH | | | | X is CCl | | | | X is CBr | | | |
| R² | R³ | R² | R³ | R² | R³ | R² | R³ | R² | R³ | R² | R³ | R² | R³ | R² | R³ |
| Cl | H | Br | H | Cl | H | Br | H | Cl | H | Br | H | Cl | H | Br | H |
| Cl | Me | Br | Me | Cl | Me | Br | Me | Cl | Me | Br | Me | Cl | Me | Br | Me |
| Cl | Et | Br | Et | Cl | Et | Br | Et | Cl | Et | Br | Et | Cl | Et | Br | Et |
| Cl | n-Pr | Br | n-Pr | Cl | n-Pr | Br | n-Pr | Cl | n-Pr | Br | n-Pr | Cl | n-Pr | Br | n-Pr |
| Cl | i-Pr | Br | i-Pr | Cl | i-Pr | Br | i-Pr | Cl | i-Pr | Br | i-Pr | Cl | i-Pr | Br | i-Pr |
| Cl | n-Bu | Br | n-Bu | Cl | n-Bu | Br | n-Bu | Cl | n-Bu | Br | n-Bu | Cl | n-Bu | Br | n-Bu |
| Cl | i-Bu | Br | i-Bu | Cl | i-Bu | Br | i-Bu | Cl | i-Bu | Br | i-Bu | Cl | i-Bu | Br | i-Bu |
| Cl | s-Bu | Br | s-Bu | Cl | s-Bu | Br | s-Bu | Cl | s-Bu | Br | s-Bu | Cl | s-Bu | Br | s-Bu |
| Cl | t-Bu | Br | t-Bu | Cl | t-Bu | Br | t-Bu | Cl | t-Bu | Br | t-Bu | Cl | t-Bu | Br | t-Bu |

**TABLE 2**

| 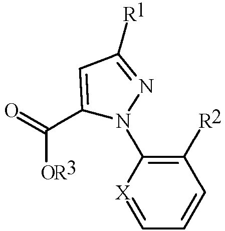 | | | | | | | | | | | | | | | |
|---|---|---|---|---|---|---|---|---|---|---|---|---|---|---|---|
| R¹ is Br | | | | | | | | | | | | | | | |
| XisN | | | | X is CH | | | | X is CCl | | | | X is CBr | | | |
| R² | R³ | R² | R³ | R² | R³ | R² | R³ | R² | R³ | R² | R³ | R² | R³ | R² | R³ |
| Cl | H | Br | H | Cl | H | Br | H | Cl | H | Br | H | Cl | H | Br | H |
| Cl | Me | Br | Me | Cl | Me | Br | Me | Cl | Me | Br | Me | Cl | Me | Br | Me |
| Cl | Et | Br | Et | Cl | Et | Br | Et | Cl | Et | Br | Et | Cl | Et | Br | Et |
| Cl | n-Pr | Br | n-Pr | Cl | n-Pr | Br | n-Pr | Cl | n-Pr | Br | n-Pr | Cl | n-Pr | Br | n-Pr |
| Cl | i-Pr | Br | i-Pr | Cl | i-Pr | Br | i-Pr | Cl | i-Pr | Br | i-Pr | Cl | i-Pr | Br | i-Pr |
| Cl | n-Bu | Br | n-Bu | Cl | n-Bu | Br | n-Bu | Cl | n-Bu | Br | n-Bu | Cl | n-Bu | Br | n-Bu |
| Cl | i-Bu | Br | i-Bu | Cl | i-Bu | Br | i-Bu | Cl | i-Bu | Br | i-Bu | Cl | i-Bu | Br | i-Bu |
| Cl | s-Bu | Br | s-Bu | Cl | s-Bu | Br | s-Bu | Cl | s-Bu | Br | s-Bu | Cl | s-Bu | Br | s-Bu |
| Cl | t-Bu | Br | t-Bu | Cl | t-Bu | Br | t-Bu | Cl | t-Bu | Br | t-Bu | Cl | t-Bu | Br | t-Bu |

### Utility

The compounds of Formulae **(I), (II**) and (A) are useful as synthetic intermediates for preparing a compound of Formula **(III)** 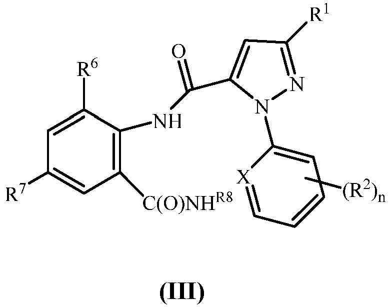 wherein X, R¹, R² and n are defined as above; R⁶ is CH₃, Cl or Br; R⁷ is CN, F, Cl, Br, I or CF₃; and R⁸ is C₁-C₄ alkyl.

Compounds of Formula **(III)** are useful as insecticides.

Compounds of Formula **(III)** can be prepared from compounds of Formula **(II)** and in turn from compounds of Formula **(A)** and **(I)** by the processes previously disclosed in US 6,965,032 B2.

## Claims

1. A method of preparing a compound of Formula (I) 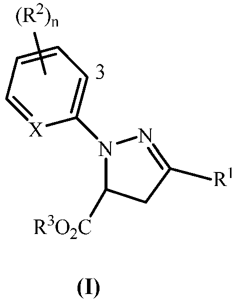
wherein R¹ is bromine;
each R² is independently C₁-C₄ alkyl, C₂-C₄ alkenyl, C₂-C₄ alkynyl, C₃-C₆ cycloalkyl, C₁-C₄ haloalkyl, C₂-C₄ haloalkenyl, C₂-C₄ haloalkynyl, C₃-C₆ halocycloalkyl, halogen, CN, NO₂, C₁-C₄ alkoxy, C₁-C₄ haloalkoxy, C₁-C₄ alkylthio, C₁-C₄ alkylsulfinyl, C₁-C₄ alkylsulfonyl, C₁-C₄ alkylamino, C₂-C₈ dialkylamino, C₃-C₆ cycloalkylamino, C₃-C₆ (alkyl)cycloalkylamino, C₂-C₄ alkylcarbonyl, C₂-C₆ alkoxycarbonyl, C₂-C₆ alkylaminocarbonyl, C₃-C₈ dialkylaminocarbonyl or C₃-C₆ trialkylsilyl;
R³ is H or C₁-C₄ alkyl;
X is N or CR₄;
R⁴ is H or R²; and
n is 0, 1, 2, or 3, with the proviso that when X is CH then n is at least 1,
the method comprising:
(a) treating a compound of Formula (A) 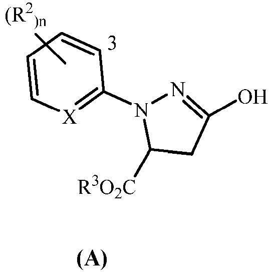
wherein R³ is C₁-C₄ alkyl;
with a phosphorus tribromide;
(b) treating the product resulting from step (a) with bromine;
and, when preparing compounds of Formula (I) wherein R³ is H, converting the compound formed in (b) to a compound wherein R³ is H;
optionally further comprising isolating the compound of Formula (I).

2. The method of claim 1 wherein the compound of Formula (I) is ethyl 3-bromo-1-(3-chloropyridin-2-yl)-4,5-dihydro-1*H*-pyrazole-5-carboxylate, having the following structure: 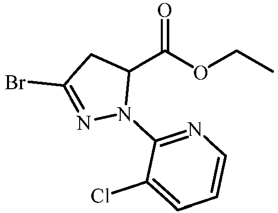 and the compound of Formula (A) is ethyl 1-(3-chloropyridin-2-yl)-3-hydroxy-4,5-dihydro-1H-pyrazole-5-carboxylate, having the structure:

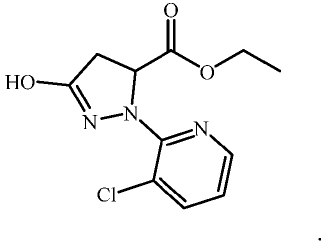

3. The method of claim 1 or claim 2, wherein the steps (a) and (b) are independently carried out at the temperature from 15 °C to 50°C.

4. The method of any one of claims 1-3 wherein the method is carried out in presence of a solvent.

5. The method of claim 4 wherein the solvent is selected form acetonitrile, N,N-dimethylformamide, dimethylacetamide, chloroform, acetone, propionitrile, chlorobenzene, tetrachloromethane, dichlorobenzene, dichloromethane , and 1,2-dichloroethane, preferably wherein the solvent is selected from acetonitrile, chlorobenzene, dichloromethane, and 1,2-dichloroethane, more preferably wherein the solvent is acetonitrile.

6. The method of any of claims 1-5, further comprising:
(c) treating the product resulting from step (b) with a base.

7. The method of claim 6 wherein the base is selected from solid sodium bicarbonate, sodium bicarbonate, potassium bicarbonate, calcium bicarbonate, sodium carbonate, potassium carbonate, ammonium carbonate, ammonium bicarbonate, trisodium phosphate, tripotassium phosphate, caesium carbonate, triethylamine, pyridine, N-methylimidazole, potassium hydrogen phosphate, sodium hydrogen phosphate, sodium hydroxide, and potassium hydroxide, preferably wherein the base is selected from solid sodium bicarbonate, saturated sodium bicarbonate, sodium carbonate, and potassium carbonate.

8. The method of claim 6 wherein the base is solid sodium bicarbonate.

9. The method of claim 8 further comprising adding water after treating the product resulting from step (b) with the solid sodium bicarbonate.

10. The method of claim 6 wherein the base is a saturated sodium bicarbonate solution.

11. The method of claim 10 further comprising adding solid sodium bicarbonate after treating the product resulting from step (b) with the saturated sodium bicarbonate solution.

12. The method of claim 6 wherein the base is a sodium carbonate solution or a potassium carbonate solution.

13. A method according to any one of claims 1-12 wherein the compound of Formula (I) is oxidized to form a compound of Formula (**II**) 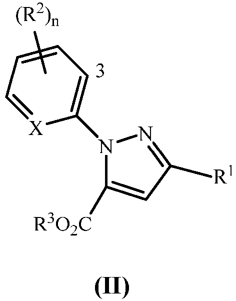
wherein R¹ is bromine;
each R² is independently C₁-C₄ alkyl, C₂-C₄ alkenyl, C₂-C₄ alkynyl, C₃-C₆ cycloalkyl, C₁-C₄ haloalkyl, C₂-C₄ haloalkenyl, C₂-C₄ haloalkynyl, C₃-C₆ halocycloalkyl, halogen, CN, NO₂, C₁-C₄ alkoxy, C₁-C₄ haloalkoxy, C₁-C₄ alkylthio, C₁-C₄ alkylsulfinyl, C₁-C₄ alkylsulfonyl, C₁-C₄ alkylamino, C₂-C₈ dialkylamino, C₃-C₆ cycloalkylamino, C₃-C₆ (alkyl)cycloalkylamino, C₂-C₄ alkylcarbonyl, C₂-C₆ alkoxycarbonyl, C₂-C₆ alkylaminocarbonyl, C₃-C₈ dialkylaminocarbonyl or C₃-C₆ trialkylsilyl;
R³ is H or C₁-C₄ alkyl;
X is N or CR₄;
R⁴ is H or R²; and n is 0, 1, 2, or 3, with the proviso that when X is CH then n is at least 1;
the method optionally further comprising isolating the compound of Formula **(II)**.

14. The method of claim 13 wherein the compound of Formula **(II)** is ethyl 3-bromo-1-(3-chloro-2-pyridinyl)-1H-pyrazole-5-carboxylate, having the following structure:

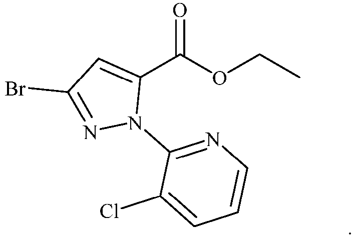

15. The method of any of claims 1 or 3-13 wherein n is 1, 2, or 3.

16. The method of any of claims 1, 3-13 or 15 wherein R² is independently Cl or Br.

17. The method of claim 16 wherein one R² is at the 3-position.

18. The method of any of claims 1, 3-13 or 15-17 wherein R³ is C₁-C₄ alkyl.

19. The method of any of claims 1, 3-13 or 15-18 wherein X is N.

## Patentansprüche

1. Verfahren zur Herstellung einer Verbindung der Formel (I) 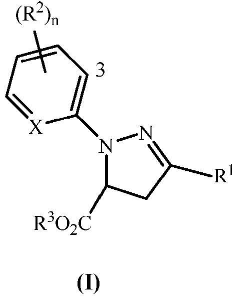
wobei R¹ für Brom steht;
R² jeweils unabhängig für C₁-C₄-Alkyl, C₂-C₄-Alkenyl, C₂-C₄-Alkinyl, C₃-C₆-Cycloalkyl, C₁-C₄-Halogenalkyl, C₂-C₄ Halogenalkenyl, C₂-C₄ Halogenalkinyl, C₃-C₆ Halogencycloalkyl, Halogen, CN, NO₂, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylamino, C₂-C₈-Dialkylamino, C₃-C₆-Cycloalkylamino, C₃-C₆ (Alkyl)cycloalkylamino, C₂-C₄-Alkylcarbonyl, C₂-C₆-Alkoxycarbonyl, C₂-C₆-Alkylaminocarbonyl, C₃-C₈-Dialkylaminocarbonyl oder C₃-C₆-Trialkylsilyl steht;
R³ für H oder C₁-C₄-Alkyl steht;
X für N oder CR₄ steht;
R⁴ für H oder R² steht; und
n für 0, 1, 2 oder 3 steht, mit der Maßgabe, dass dann, wenn X für CH steht, n mindestens gleich 1 ist,
wobei das Verfahren Folgendes umfasst:
(a) Behandeln einer Verbindung der Formel (A) 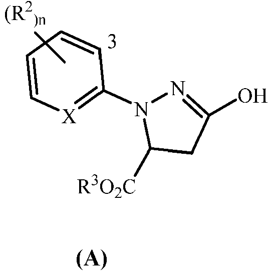
wobei R³ für C₁-C₄-Alkyl steht;
mit einem Phosphortribromid;
b) Behandeln des aus Schritt (a) resultierenden Produkts mit Brom;
und bei der Herstellung von Verbindungen der Formel (I), in denen R³ für H steht, Umwandeln der in (b) gebildeten Verbindung in eine Verbindung, in der R³ für H steht;
gegebenenfalls ferner umfassend das Isolieren der Verbindung der Formel (I).

2. Verfahren nach Anspruch 1, wobei es sich bei der Verbindung der Formel (I) um 3-Brom-1-(3-chlorpyridin-2-yl)-4,5-dihydro-1H-pyrazol-5-carbonsäureethylester mit der folgenden Struktur handelt: 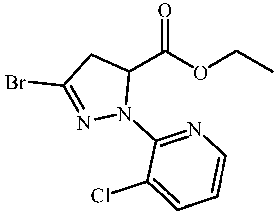 und es sich bei der Verbindung der Formel (A) um 1-(3-Chlorpyridin-2-yl)-3-hydroxy-4,5-dihydro-1H-pyrazol-5-carbonsäureethylester mit der folgenden Struktur handelt:

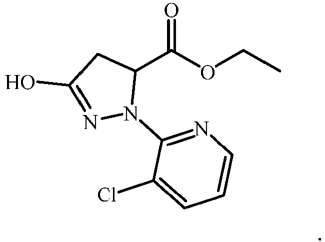

3. Verfahren nach Anspruch 1 oder Anspruch 2, wobei die Schritte (a) und (b) unabhängig bei der Temperatur von 15 °C bis 50 °C durchgeführt werden.

4. Verfahren nach einem der Ansprüche 1-3, wobei das Verfahren in Gegenwart eines Lösungsmittels durchgeführt wird.

5. Verfahren nach Anspruch 4, wobei das Lösungsmittel aus Acetonitril, N,N-Dimethylformamid, Dimethylacetamid, Chloroform, Aceton, Propionitril, Chlorbenzol, Tetrachlormethan, Dichlorbenzol, Dichlormethan und 1,2-Dichlorethan ausgewählt wird, vorzugsweise wobei das Lösungsmittel ist aus Acetonitril, Chlorbenzol, Dichlormethan und 1,2-Dichlorethan ausgewählt wird, weiter bevorzugt wobei es sich bei dem Lösungsmittel um Acetonitril handelt.

6. Verfahren nach einem der Ansprüche 1-5, ferner umfassend:
(c) Behandeln des aus Schritt (b) resultierenden Produkts mit einer Base.

7. Verfahren nach Anspruch 6, wobei die Base aus festem Natriumhydrogencarbonat, Natriumhydrogencarbonat, Kaliumhydrogencarbonat, Calciumhydrogencarbonat, Natriumcarbonat, Kaliumcarbonat, Ammoniumcarbonat, Ammoniumhydrogencarbonat, Trinatriumphosphat, Trikaliumphosphat, Caesiumcarbonat, Triethylamin, Pyridin, N-Methylimidazol, Kaliumhydrogenphosphat, Natriumhydrogenphosphat, Natriumhydroxid und Kaliumhydroxid ausgewählt wird, vorzugsweise wobei die Base aus festem Natriumhydrogencarbonat, gesättigtem Natriumhydrogencarbonat, Natriumcarbonat und Kaliumcarbonat ausgewählt wird.

8. Verfahren nach Anspruch 6, wobei es sich bei der Base um festes Natriumhydrogencarbonat handelt.

9. Verfahren nach Anspruch 8, ferner umfassend das Zugeben von Wasser nach dem Behandeln des aus Schritt (b) resultierenden Produkts mit dem festen Natriumhydrogencarbonat.

10. Verfahren nach Anspruch 6, wobei es sich bei der Base um eine gesättigte Natriumhydrogencarbonatlösung handelt.

11. Verfahren nach Anspruch 10, ferner umfassend das Zugeben von festem Natriumhydrogencarbonat nach dem Behandeln des aus Schritt (b) resultierenden Produkts mit der gesättigten Natriumhydrogencarbonatlösung.

12. Verfahren nach Anspruch 6, wobei es sich bei der Base um eine Natriumcarbonatlösung oder eine Kaliumcarbonatlösung handelt.

13. Verfahren nach einem der Ansprüche 1-12, wobei die Verbindung der Formel (I) zu einer Verbindung der Formel (II) oxidiert wird: 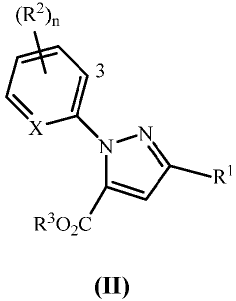
wobei R¹ für Brom steht;
R² jeweils unabhängig für C₁-C₄-Alkyl, C₂-C₄-Alkenyl, C₂-C₄-Alkinyl, C₃-C₆-Cycloalkyl, C₁-C₄-Halogenalkyl, C₂-C₄ Halogenalkenyl, C₂-C₄ Halogenalkinyl, C₃-C₆ Halogencycloalkyl, Halogen, CN, NO₂, C₁-C₄-Alkoxy, C₁-C₄-Halogenalkoxy, C₁-C₄-Alkylthio, C₁-C₄-Alkylsulfinyl, C₁-C₄-Alkylsulfonyl, C₁-C₄-Alkylamino, C₂-C₈-Dialkylamino, C₃-C₆-Cycloalkylamino, C₃-C₆ (Alkyl)cycloalkylamino, C₂-C₄-Alkylcarbonyl, C₂-C₆-Alkoxycarbonyl, C₂-C₆-Alkylaminocarbonyl, C₃-C₈-Dialkylaminocarbonyl oder C₃-C₆-Trialkylsilyl steht;
R³ für H oder C₁-C₄-Alkyl steht;
X für N oder CR₄ steht;
R⁴ für H oder R² steht; und n für 0, 1, 2 oder 3 steht, mit der Maßgabe, dass dann, wenn X für CH steht, n mindestens gleich 1 ist;
wobei das Verfahren gegebenenfalls ferner das Isolieren der Verbindung der Formel (II) umfasst.

14. Verfahren nach Anspruch 13, wobei es sich bei der Verbindung der Formel (II) um 3-Brom-1-(3-chlor-2-pyridinyl)-1H-pyrazol-5-carbonsäureethylester mit der folgenden Struktur handelt:

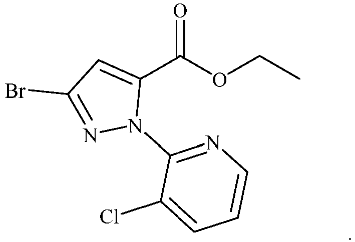

15. Verfahren nach einem der Ansprüche 1 oder 3-13, wobei n für 1, 2 oder 3 steht.

16. Verfahren nach einem der Ansprüche 1, 3-13 oder 15, wobei R² unabhängig für Cl oder Br steht.

17. Verfahren nach Anspruch 16, wobei sich ein R² in der 3-Position befindet.

18. Verfahren nach einem der Ansprüche 1, 3-13 oder 15-17, wobei R³ für C₁-C₄-Alkyl steht.

19. Verfahren nach einem der Ansprüche 1, 3-13 oder 15-18, wobei X für N steht.

## Revendications

1. Procédé de préparation d'un composé de formule (I) 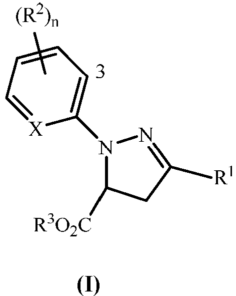
R¹ étant brome ;
chaque R² étant indépendamment C₁-C₄ alkyle, C₂-C₄ alcényle, C₂-C₄ alcynyle, C₃-C₆ cycloalkyle, C₁-C₄ halogénoalkyle, C₂-C₄ halogénoalcényle, C₂-C₄ halogénoalcynyle, C₃-C₆ halogénocycloalkyle, halogène, CN, NO₂, C₁-C₄ alcoxy, C₁-C₄ halogénoalcoxy, C₁-C₄ alkylthio, C₁-C₄ alkylsulfinyle, C₁-C₄ alkylsulfonyle, C₁-C₄ alkylamino, C₂-C₈ dialkylamino, C₃-C₆ cycloalkylamino, C₃-C₆ (alkyl)cycloalkylamino, C₂-C₄ alkylcarbonyle, C₂-C₆ alcoxycarbonyle, C₂-C₆ alkylaminocarbonyle, C₃-C₈ dialkylaminocarbonyle ou C₃-C₆ trialkylsilyle ;
R³ étant H ou C₁-C₄ alkyle ;
X étant N ou CR₄ ;
R⁴ étant H ou R² ; et
n étant 0, 1, 2, ou 3, à condition que lorsque X est CH alors n est au moins 1,
le procédé comprenant :
(a) le traitement d'un composé de formule (A) 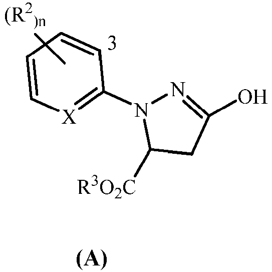
R³ étant C₁-C₄ alkyle ;
avec un tribromure de phosphore ;
(b) le traitement du produit résultant de l'étape (a) avec du brome ;
et, lors de la préparation de composés de formule (I) dans laquelle R³ est H, la conversion du composé formé en (b) en un composé dans lequel R³ est H ;
éventuellement comprenant en outre l'isolement du composé de formule (I).

2. Procédé selon la revendication 1, dans lequel le composé de formule (I) est le 3-bromo-1-(3-chloropyridin-2-yl)-4,5-dihydro-1H-pyrazole-5-carboxylate d'éthyle, ayant la structure suivante : 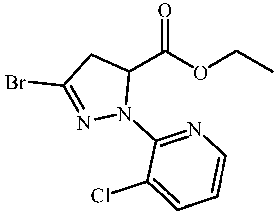 et le composé de formule (A) est le 1-(3-chloropyridin-2-yl)-3-hydroxy-4,5-dihydro-1H-pyrazole-5-carboxylate d'éthyle, ayant la structure :

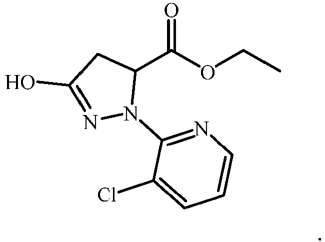

3. Procédé selon la revendication 1 ou la revendication 2, dans lequel les étapes (a) et (b) sont réalisées indépendamment à la température de 15 °C à 50 °C.

4. Procédé selon l'une quelconque des revendications 1-3 dans lequel le procédé est mis en œuvre en présence d'un solvant.

5. Procédé selon la revendication 4, dans lequel le solvant est choisi parmi l'acétonitrile, le N,N-diméthylformamide, le diméthylacétamide, le chloroforme, l'acétone, le propionitrile, le chlorobenzène, le tétrachlorométhane, un dichlorobenzène, le dichlorométhane, et le 1,2-dichloroéthane, de préférence dans lequel le solvant est choisi parmi l'acétonitrile, le chlorobenzène, le dichlorométhane et le 1,2-dichloroéthane, plus préférablement dans lequel le solvant est l'acétonitrile.

6. Procédé selon l'une quelconque des revendications 1 à 5, comprenant en outre :
(c) le traitement du produit résultant de l'étape (b) avec une base.

7. Procédé selon la revendication 6, dans lequel la base est choisie parmi le bicarbonate de sodium solide, le bicarbonate de sodium, le bicarbonate de potassium, le bicarbonate de calcium, le carbonate de sodium, le carbonate de potassium, le carbonate d'ammonium, le bicarbonate d'ammonium, le phosphate trisodique, le phosphate tripotassique, le carbonate de césium, la triéthylamine, la pyridine, le N-méthylimidazole, l'hydrogénophosphate de potassium, l'hydrogénophosphate de sodium, l'hydroxyde de sodium et l'hydroxyde de potassium, de préférence dans lequel la base est choisie parmi le bicarbonate de sodium solide, le bicarbonate de sodium saturé, le carbonate de sodium et le carbonate de potassium.

8. Procédé selon la revendication 6, dans lequel la base est le bicarbonate de sodium solide.

9. Procédé selon la revendication 8, comprenant en outre l'ajout d'eau après le traitement du produit résultant de l'étape (b) avec le bicarbonate de sodium solide.

10. Procédé selon la revendication 6, dans lequel la base est une solution saturée de bicarbonate de sodium.

11. Procédé selon la revendication 10, comprenant en outre l'ajout de bicarbonate de sodium solide après traitement du produit résultant de l'étape (b) avec la solution saturée de bicarbonate de sodium.

12. Procédé selon la revendication 6, dans lequel la base est une solution de carbonate de sodium ou une solution de carbonate de potassium.

13. Procédé selon l'une quelconque des revendications 1-12 dans lequel le composé de formule (I) est oxydé pour former un composé de formule (II) 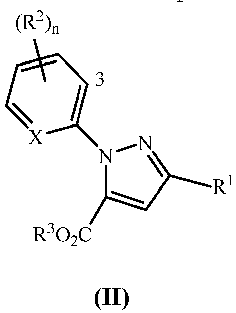
R¹ étant brome ;
chaque R² étant indépendamment C₁-C₄ alkyle, C₂-C₄ alcényle, C₂-C₄ alcynyle, C₃-C₆ cycloalkyle, C₁-C₄ halogénoalkyle, C₂-C₄ halogénoalcényle, C₂-C₄ halogénoalcynyle, C₃-C₆ halogénocycloalkyle, halogène, CN, NO₂, C₁-C₄ alcoxy, C₁-C₄ halogénoalcoxy, C₁-C₄ alkylthio, C₁-C₄ alkylsulfinyle, C₁-C₄ alkylsulfonyle, C₁-C₄ alkylamino, C₂-C₈ dialkylamino, C₃-C₆ cycloalkylamino, C₃-C₆ (alkyl) cycloalkylamino, C₂-C₄ alkylcarbonyle, C₂-C₆ alcoxycarbonyle, C₂-C₆ alkylaminocarbonyle, C₃-C₈ dialkylaminocarbonyle ou C₃-C₆ trialkylsilyle ;
R³ étant H ou C₁-C₄ alkyle ;
X étant N ou CR₄ ;
R⁴ étant H ou R² ; et n étant 0, 1, 2, ou 3, à condition que lorsque X est CH alors n est au moins 1 ;
le procédé comprenant éventuellement en outre l'isolement du composé de formule (II).

14. Procédé selon la revendication 13, dans lequel le composé de formule (II) est le 3-bromo-1-(3-chloro-2-pyridinyl)-1H-pyrazole-5-carboxylate d'éthyle, ayant la structure suivante :

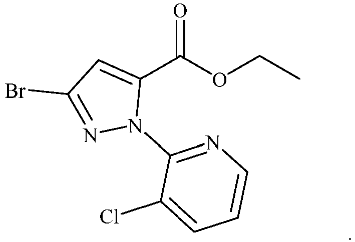

15. Procédé selon l'une quelconque des revendications 1 ou 3-13, dans lequel n est 1, 2 ou 3.

16. Procédé selon l'une quelconque des revendications 1, 3-13 ou 15, dans lequel R² est indépendamment Cl ou Br.

17. Procédé selon la revendication 16, dans lequel un R² est au niveau de la position 3.

18. Procédé selon l'une quelconque des revendications 1, 3-13 ou 15-17, dans lequel R³ est C₁-C₄ alkyle.

19. Procédé selon l'une quelconque des revendications 1, 3-13 ou 15-18, dans lequel X est N.
